Europäisches Patentamt

European Patent Office

Office européen des brevets

(19)

(11) Veröffentlichungsnummer : **0 136 664**
**B1**

(12)

# EUROPÄISCHE PATENTSCHRIFT

(45) Veröffentlichungstag der Patentschrift :
03.01.90

(21) Anmeldenummer : 84111569.4

(22) Anmeldetag : 27.09.84

(51) Int. Cl.⁵ : **G 01 N 1/22**, G 01 N 33/18,
B 01 D 19/00

(54) Kontaktapparat für Gasanalysator.

(30) Priorität : 06.10.83 DE 3336423

(43) Veröffentlichungstag der Anmeldung :
10.04.85 Patentblatt 85/15

(45) Bekanntmachung des Hinweises auf die Patenterteilung : 03.01.90 Patentblatt 90/01

(84) Benannte Vertragsstaaten :
AT BE CH DE FR GB IT LI LU NL SE

(56) Entgegenhaltungen :
DE-A- 2 363 370
FR-A- 1 126 521
FR-A- 1 457 388

(73) Patentinhaber : INTERATOM Gesellschaft mit beschränkter Haftung
Friedrich-Ebert-Strasse
D-5060 Bergisch-Gladbach 1 (DE)

(72) Erfinder : Ebbers, Gottfried
Bensberger Strasse 1
D-5063 Overath 3 (DE)
Erfinder : Zschetke, Jürgen
Eichweiler 12
D-5067 Kürten (DE)

(74) Vertreter : Mehl, Ernst, Dipl.-Ing. et al
Postfach 22 13 17
D-8000 München 22 (DE)

Beschreibung

Die vorliegende Erfindung betrifft einen Kontaktapparat für einen Gasanalysator zur quasi kontinuierlichen Messung von geringen Gasanteilen in einem kontinuierlichen Wasserstrom. Mit einem Wärmeleitfähigkeitsdetektor und Argon als Trägergas ergibt sich für Wasserstoff eine Nachweisgrenze von 0,01 ppb, für Sauerstoff 0,5 ppb und für Stickstoff 1 ppb. Übliche Meßwerte an Kraftwerken liegen zwischen 1 und einigen 100 ppb, bei der Überwachung eines Verfahrens zur katalytischen Sauerstoffentfernung treten auch Werte im Prozentbereich auf.

Auf der Grundlage des HENRY-DALTON'schen Gesetzes wird das kontinuierlich fließende Probenwasser mit einem Trägergas unter genau definierten Bedingungen in Kontakt gebracht, wobei das Trägergas, z. B. hochreines Argon, die im Probenwasser vorhandenen Gase mitnimmt. Nach Entfernen der im Trägergas aufgenommenen Feuchte wird das angereicherte Gasgemisch mit einer mit einem Molekularsieb gefüllten Trennsäule chromatographisch getrennt und anschließend quantitativ über Wärmeleitfähigkeitsdetektion erfaßt. Die vorliegende Erfindung betrifft den Kontaktapparat, in dem das Trägergas mit dem Probenwasser in Berührung gebracht wird. Eine Vorrichtung dieser Art ist bereits in der deutschen Offenlegungsschrift 23 63 370.7 beschrieben.

Aufgabe der vorliegenden Erfindung ist es, die Empfindlichkeit und die Meßgenauigkeit des bekannten Gerätes zu steigern. Für die Geräte dieser Art ist eine weitgehend konstante Temperatur von Probenwasser und Trägergas von Bedeutung und die Zusammenführung und Trennung von Probenwasser und Trägergas muß sehr sorgfältig und unter konstanten Bedingungen vorgenommen werden. Bei Zu- und Abfluß von Probenwasser und Trägergas, wie auch im eigentlichen Kontaktrohr sollen Gasblasen vermieden werden, die zu Druckschwankungen und damit auch zu Durchflußänderungen führen können und die Feuchtigkeit in die anschließende Trennsäule transportieren könnten.

Zur Lösung dieser Aufgaben wird zunächst ein Kontaktapparat nach dem ersten Anspruch vorgeschlagen. Die vorgesehene Anordnung in einem Behälter, der während des Betriebes mit einer wärmeleitenden Flüssigkeit, z. B. Wasser gefüllt ist, ergibt eine weitgehend konstante Temperatur von Probenwasser und Trägergas. Selbst kleine, unvermeidliche Temperaturunterschiede zwischen Trägergas und Probenwasser werden durch die vorgesehene schraubenlinienartige Anordnung nach dem Gegenstromprinzip ausgeglichen. Der innerhalb der beiden Rohrschlangen angeordnete Zylinder mit seinen Öffnungen am oberen und unteren Ende und mit dem darin enthaltenen Heizelement ergibt aufgrund einer Konvektion einen ständigen Umlauf des Wassers im Behälter, das innerhalb des Zylinders beheizt wird und dementsprechend aufsteigt und außerhalb des Zylinders abwärts strömt. Durch die vorgeschlagene Anordnung des Heizelementes innerhalb des Zylinders wird vermieden, daß einzelne heiße Strähnen das Kontaktrohr direkt berühren und dort zeitlich und örtlich unterschiedliche Temperaturen hervorrufen.

In weiterer Ausgestaltung der Erfindung wird im Anspruch 2 vorgeschlagen, auch die Zusammenführung und Trennung von Probenwasser und Trägergas innerhalb des thermostatisierten Behälters anzuordnen. Auch damit wird die Einhaltung einer konstanten Temperatur verbessert. Bisher wurden die Anschlüsse für Probenwasser und Trägergas außerhalb des thermostatisierten Behälters zusammengeführt, damit man diese, für die störungsfreie Funktion sehr wichtigen Teile prüfen und ihre Anschlüsse gegebenenfalls auswechseln kann. Für eine genaue Messung hat es sich aber als bedeutsam herausgestellt, daß auch diese Teile thermostatisiert sind.

Mit den abhängigen Ansprüchen werden weitere Ausgestaltungen der Vorrichtung vorgeschlagen, die sich im Versuch bewährt haben und mit denen ein konstanter Durchfluß von Probenwasser und Trägergas innerhalb des Kontaktapparates gewährleistet werden kann.

Die Figuren 1 bis 4 zeigen ein mögliches Ausführungsbeispiel der Erfindung.

Fig. 1 zeigt einen senkrechten Längsschnitt durch den in einem zylindrischen Behälter angeordneten Kontaktapparat.

Fig. 2 zeigt den im unteren Teil des Behälters angeordneten Trennblock.

Fig. 3 zeigt den im oberen Teil des Behälters angeordneten Trennblock.

Fig. 4 zeigt einen gasdichten Wasserabfluß.

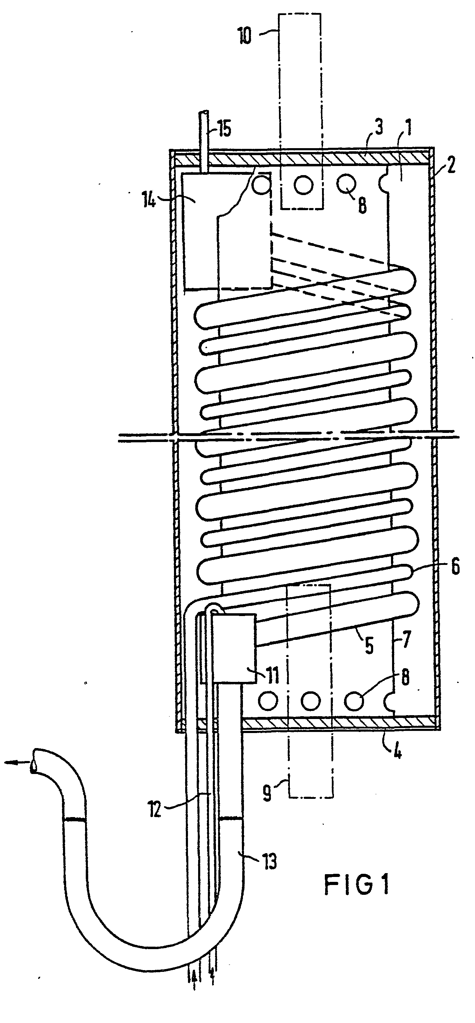

In Fig. 1 enthält der Behälter 1, bestehend aus einem zylindrischen Teil 2, einem Deckel 3 und einem Boden 4, zwei schraubenlinienartig angeordnete Rohre und zwar das Kontaktrohr 5 und von wesentlich geringerem Durchmesser, das Wasserzufuhrrohr 6, die beide mit Abstand um einen inneren Zylinder 7 gewickelt sind, der an Boden 4 und Deckel 3 anliegt und dort jeweils mehrere, gleichmäßig über den Umfang verteilte radiale Öffnungen 8 aufweist.

Im Boden 4 und zwar innerhalb des Zylinders 7 ist ein elektrisches Heizelement 9 zentral und dicht eingesetzt, das von einem Temperaturmeßgerät 10, das im Deckel 3 dicht und zentral eingesetzt ist, geregelt wird. Das untere Ende des Kontaktrohres 5 ist in einen Trennblock 11 eingeschweißt, der außerdem eine Gaszufuhrleitung 12 und eine Wasserabflußleitung 13 trägt, die mit einem U-Bogen endet. Die Wasserzufuhrleitung 6 ist mit ihrem oberen Ende wie auch das Kontaktrohr 5 in einem Trennblock 14 eingeschweißt, der noch eine Gasabfuhrleitung 15 trägt, die nach oben aus dem Behälter 1 herausführt.

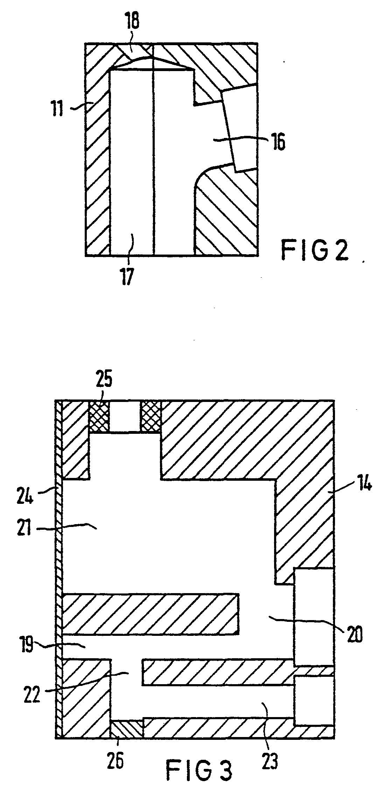

Fig. 2 zeigt den unteren Trennblock 11 aus Fig. 1, mit dem das aus dem Kontaktrohr 5 austretende Probenwasser zu einem Abfluß geleitet wird und mit dem das Trägergas durch Bohrung (18) in das Kontaktrohr eingeleitet wird. An diesem Trennblock ist von Bedeutung, daß das Trägergas oberhalb des Probenwassers eintritt, so daß keine Gasblasen im Wasserstrom entstehen können. Weiterhin soll der Übergang zwischen der Bohrung 16 und der Bohrung 17 abgerundet sein, damit dort das Probenwasser laminar und ohne Tropfenbildung abfließen kann. Damit diese Abrundung bearbeitet und geprüft werden kann, wird der Trennblock 11 zweckmäßigerweise vor dem Bearbeiten in zwei Teile getrennt entsprechend der schwarzen Linie in Bohrung 17 und erst nach der Bearbeitung verlötet.

Fig. 3 zeigt den Trennblock 14 im Längsschnitt. Bei der Herstellung dieses Trennblockes werden zunächst die im Anspruch 4 beschriebenen Bohrungen angebracht und anschließend, wie in der Fig. 3 dargestellt, durch Schweissen oder Auflöten einer Platte 24 geschlossen. Das zufließende Probenwasser ohne Trägergas wird durch die Bohrungen 23 und 22 zur Bohrung 19 geleitet, die es am unteren Ende in das an die Bohrung 20 angeschlossene Kontaktrohr 5 einmünden läßt. Durch diese Anordnung wird vermieden, daß innerhalb des Trennblocks 14 Gasblasen entstehen, die zu kleinen Druckschwankungen oder zu einem Transport von Feuchtigkeit in die anschließende Trennsäule führen könnten. Oberhalb der Bohrungen 19 und 20 ist ein Gassammelraum 21 angeordnet, der ebenfalls einen Transport von Feuchtigkeit mit dem Trägergasstrom verhindern soll. Am oberen Ende dieses Gassammelraumes 21 ist ein Reduzierstück 25 für den Anschluß der Gasabführleitung 15 angeordnet. Am unteren Ende ist die Bohrung 22 im Trennblock 14 durch einen Verschlußstopfen 26 geschlossen.

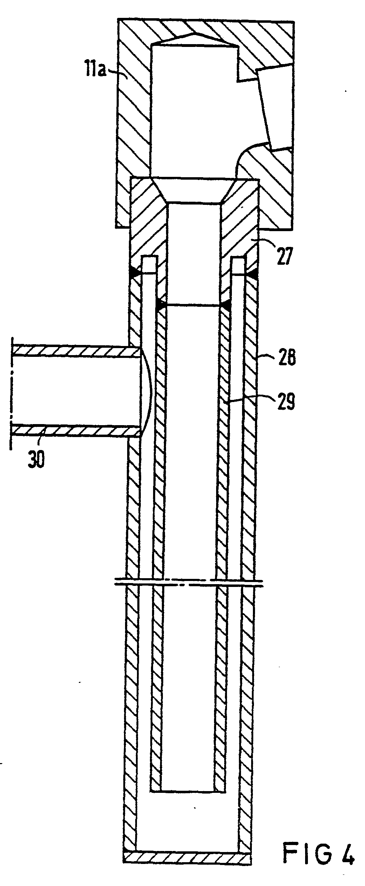

Fig. 4 zeigt als Alternative zu dem in Fig. 1 dargestellten U-Bogen der Wasserabflußleitung 13 einen ebenfalls gasdichten Wasserabfluß 27, der ebenfalls am unteren Ende eines Trennblockes 11 nach Fig. 2 angeschlossen sein kann. Sowohl der U-Bogen als auch der Wasserabfluß 27 sollen in einem Raum von Atmosphärendruck enden, damit der Druck im Kontaktrohr 5 konstant bleibt, und sollen außerdem das Kontaktrohr gegen den Eintritt unerwünschter Gasmengen schützen. Der Abfluß 27 besteht aus mehreren miteinander verschweißten Rohrstutzen und Drehteilen, deren Gestaltung aus der Fig. 4 ersichtlich ist.

## Patentansprüche

1. Kontaktapparat für einen Gasanalysator zur kontinuierlichen Messung von geringen Gasanteilen in Wasser, bestehend aus einem thermostatisierten Behälter mit einem schraubenlinienartig gewundenen Kontaktrohr, in dem das Probenwasser abwärts und im Gegenstrom dazu ein Trägergas aufwärts strömen kann, dadurch gekennzeichnet, daß das in den Behälter (1) eintretende Rohr (6) für Probenwasser ebenfalls schraubenlinienartig und zwar mit Abstand zum Kontaktrohr (5) geführt ist und das innerhalb der beiden Rohre ein Zylinder (7) angeordnet ist mit mehreren radialen Öffnungen (8) an seinem oberen und unteren Ende und daß dieser Zylinder in seinem unteren Ende ein Heizelement (9) enthält.

2. Kontaktapparat nach Anspruch 1, dadurch gekennzeichnet, daß innerhalb des Behälters (1) am unteren Ende des Kontaktrohres (5) ein unterer Trennblock (11) und am oberen Ende des Kontaktrohres (5) ein oberer Trennblock (14) für Probenwasser und Trägergas angeordnet ist.

3. Kontaktapparat nach Anspruch 2, dadurch gekennzeichnet, daß der untere Trennblock (11) eine seitliche Bohrung (16) für den Anschluß des unteren Endes des Kontaktrohres (5) und eine nach unten gerichtete Bohrung (17) für den Abfluß des Probenwassers trägt und am oberen Ende des Trennblockes eine nach unten gerichtete Bohrung (18) für die Gaszufuhrleitung (12) des Trägergases angeordnet ist.

4. Kontaktapparat nach Anspruch 2, dadurch gekennzeichnet, daß innerhalb des oberen Trennblockes (14) eine annähernd waagerechte Bohrung (19) für das Probenwasser ohne Trägergas angeordnet ist, deren Durchmesser wesentlich kleiner ist als der des Kontaktrohres (5) und die am untersten Punkt der Bohrung (20) für das Kontaktrohr (5) mündet und daß oberhalb dieser beiden Bohrungen ein Gassammelraum (21) angeordnet ist, der an seinem oberen Ende an die Leitung (15) für das ausfließende Trägergas anzuschließen ist.

5. Kontaktapparat nach Anspruch 4, dadurch gekennzeichnet, daß unterhalb der genannten Bohrungen (19 und 20) zwei weitere Bohrungen (22 und 23) angeordnet sind, die das Probenwasser von dem schraubenlinienartig angeordneten Rohr (6) zu der in das Kontaktrohr (5) einmündenden Bohrung leiten.

6. Kontaktapparat nach Anspruch 3, dadurch gekennzeichnet, daß am unteren Ende des Trennblocks (11a) ein Abfluß 27 angeordnet ist, der mit zwei konzentrischen Rohren (28, 29) am unteren Ende verbunden ist, wobei das äußere Rohr (28) unten verschlossen ist und an seinem oberen Ende einen seitlichen Rohrstutzen (30) trägt.

## Claims

1. Contacting device for an analyzer for continual measurement of small portions of gas in water, consisting of a thermo-statisized container with a spirally wound contact tube in which the test water can flow downwards and a carrier gas can flow upwards in counter flow thereto, characterised in that the tube for the test water entering into the container (1) is also led in a spiral manner and spaced from the contact tube (5) and that a cylinder (7) is arranged within both tubes with several radial openings (8) on its upper and lower end and that this cylinder contains a heating element (9) in its lower end.

2. Contacting device according to claim 1, characterised in that arranged within the container (1) at the lower end of the contact tube (5) there is a lower separating block (11) and at the upper end of the contact tube (5) an upper separating block (14) for the test water and carrier gas.

3. Contacting device according to claim 2, characterised in that the lower separating block (11) carries a lateral bore (16) for the connection of the lower end of the contact tube (5) and a bore (17) directed downwards for the discharge of the test water and arranged at the upper end of the separating block there is a bore (18) directed downwards for the gas supply line (12) of the carrier gas.

4. Contacting device according to claim 2, characterised in that arranged within the upper separating block (14) there is an approximately horizontal bore (19) for the test water without carrier gas, the diameter of which is substantially smaller than that of the contact tube (5) and which opens at the lowest point of the bore (20) for the contact tube (5) and that above both these bores there is arranged a gas-collecting space (21) which is to be attached at its upper end to the line (15) for the carrier gas flowing out.

5. Contacting apparatus according to claim 4, characterised in that arranged below the named bores (19 and 20) there are two further bores (22 and 23) which lead the test water from the spirally arranged tube to the bore opening into the contact tube (5).

6. Contacting apparatus according to claim 3, characterised in that on the lower end of the separating block (11a) there is arranged an outlet (27) which is connected with two concentric tubes (28, 29) at the lower end, wherein the outer tube (28) is sealed at the bottom and at its upper end carries a lateral tube socket (30).

**Revendications**

1. Appareil de mise en contact, pour un analyseur de gaz, en vue de mesurer en continue de faibles proportions de gaz dans de l'eau, constitué d'une cuve thermostatisée, ayant un tube de mise en contact qui est enroulé en hélice et dans lequel peuvent s'écouler vers le bas l'eau à analyser, et vers le haut et à contre-courant un gaz porteur, caractérisé en ce que, le tuyau (6), qui pénètre dans la cuve (1) et qui est destiné à l'eau à analyser, est guidé également hélicoïdale- ment et à distance du tuyau de mise en contact (5) et en ce que, à l'intérieur des deux tuyaux est disposé un cylindre (7) ayant plusieurs orifices radiaux (8) à son extrémité supérieure et à son extrémité inférieure et en ce que ce cylindre comporte à son extrémité inférieure un élément de chauffage (9).

2. Appareil de mise en contact suivant la revendication 1, caractérisé en ce que, à l'intérieur de la cuve (1), est monté, à l'extrémité supérieure du tuyau de mise en contact (5), un bloc inférieur de séparation (11) et, à l'extrémité supérieure du tuyau de mise en contact (5), un bloc supérieur de séparation (14), pour l'eau à analyser et pour le gaz porteur.

3. Appareil de mise en contact suivant la revendication 2, caractérisé en ce que, le bloc inférieur de séparation (11) porte un perçage latéral (16) pour le raccordement de l'extrémité inférieure du tuyau de mise en contact (5) et un perçage (17) dirigé vers le bas pour l'évacuation de l'eau à analyser et, à l'extrémité supérieure du bloc de séparation, un perçage (18) qui est dirigé vers le bas et qui est destiné au conduit d'alimentation (12) en gaz porteur.

4. Appareil de mise en contact suivant la revendication 2, caractérisé en ce que, à l'intérieur du bloc supérieur de séparation (14) est prévu un perçage (19) sensiblement horizontal et destiné à l'eau à analyser, sans gaz porteur, dont le diamètre est sensiblement plus petit que celui du tuyau de mise en contact (5) et qui débouche au point le plus bas du perçage (20) pour le tuyau de mise en contact (5) et en ce que, au-dessus de ces deux perçages, est disposée une chambre collectrice de gaz (21), qui peut être raccordée à son extrémité supérieure au conduit (15) pour le gaz porteur qui sort.

5. Appareil de mise en contact suivant la revendication 4, caractérisé en ce que, en dessous desdits perçages (19 et 20) sont ménagés deux autres perçages (22 et 23) qui conduisent l'eau à analyser du tuyau (6) hélicoïdal au perçage débouchant dans le tuyau de mise en contact (5).

6. Appareil de mise en contact suivant la revendication 3, caractérisé en ce que, à l'extrémité inférieure du bloc de séparation (11a) est prévue une évacuation (27) qui communique à l'extrémité inférieure avec deux tuyaux concentriques (28, 29), le tuyau extérieur (28) étant fermé vers le bas et portant à son extrémité supérieure un raccord tubulaire (30) latéral.

FIG 1

1

FIG 2

FIG 3

FIG 4